# EUROPEAN PATENT SPECIFICATION

(11) **EP 3 887 385 B1**
(45) Date of publication and mention of the grant of the patent: **20.03.2024**
(21) Application number: 19890138.1
(22) Date of filing: 30.11.2019
(51) Int. Cl.: C07H 21/00, C12P 19/34, C40B 40/06, C40B 50/14, C40B 50/18, C12N 15/09, C12Q 1/6806

(54) **A METHOD FOR GENERATING RANDOM OLIGONUCLEOTIDES AND DETERMINING THEIR SEQUENCE**
VERFAHREN ZUR ERZEUGUNG STATISTISCHER OLIGONUKLEOTIDE UND BESTIMMUNG IHRER SEQUENZ
PROCÉDÉ DE GÉNÉRATION D'OLIGONUCLÉOTIDES ALÉATOIRES ET DE DÉTERMINATION DE LEUR SÉQUENCE

(30) Priority: 30.11.2018 US 201862773671 P
(43) Date of publication of application: 06.10.2021
(73) Proprietor: Geneinfosec, Inc., Boulder, CO 80301 (US)
(72) Inventor: SAWAYA, Sterling, Boulder, Colorado 80306 (US)
(74) Representative: Marks & Clerk LLP
(86) International application number: PCT/US2019/063890
(87) International publication number: WO 2020/113195

(56) References cited:
- WO-A1-2015/031691
- WO-A1-2016/162309
- WO-A1-2017/176541
- US-A1- 2009 170 074
- US-A1- 2016 089 651
- US-B1- 6 350 595
- FUJISHIMA KOSUKE ET AL: "An overhang-based DNA block shuffling method for creating a customized random library", SCIENTIFIC REPORTS, vol. 5, no. 1, 1 September 2015 (2015-09-01), XP055901747, DOI: 10.1038/srep09740 Retrieved from the Internet: URL:https://www.nature.com/articles/srep09 740.pdf>
- LI HUAN-HAO ET AL: "Evaluation of aptamer specificity with or without primers using clinical samples for C-reactive protein by magnetic-assisted rapid aptamer selection", RSC ADVANCES, vol. 7, no. 68, 1 January 2017 (2017-01-01), pages 42856-42865, XP055901746, DOI: 10.1039/C7RA07249J Retrieved from the Internet: URL:https://pubs.rsc.org/en/content/articl epdf/2017/ra/c7ra07249j>
- Lauren b. a. Woodruff ET AL: "Registry in a tube: multiplexed pools of retrievable parts for genetic design space exploration", Nucleic Acids Research, 21 December 2016 (2016-12-21), page gkw1226, XP055422597, GB ISSN: 0305-1048, DOI: 10.1093/nar/gkw1226
- Lim Hyeonseob ET AL: "Highly selective retrieval of accurate DNA utilizing a pool of in situ -replicated DNA from multiple next-generation sequencing platforms", Nucleic Acids Research, vol. 46, no. 7, 18 January 2018 (2018-01-18), pages 1-11, XP055896859, GB ISSN: 0305-1048, DOI: 10.1093/nar/gky016

## Description

### CROSS REFERENCE TO RELATED APPLICATION(S)

This application claims priority to co-pending U.S. Provisional Patent Application No. 62/773,671, filed on November 30, 2018.

### BACKGROUND

By combining different nucleic acids, a very large number of different oligonucleotides/polynucleotides can be generated. Currently, the synthesis of these molecules requires precision, such that the sequence of nucleotide bases present in the molecules is known prior to generating the oligonucleotide.

In some instances, a large number of random oligonucleotides are desired. For example, oligonucleotides can act as unique molecular tags, labeling individual molecules of DNA. This unique tagging is useful for some analyses, for example, tagging a molecule prior to amplification to improve the accuracy of analyses. See Zheng et al. (2014) Anchored multiplex PCR for targeted next-generation sequencing, Nature Medicine 20:1479-84. A recent invention uses unique molecular tags for cryptography (See PCT/US17/058076 application, PCT Publication WO 2018/081113 to Sawaya). This cryptography technique requires a large number of unique molecular tags to be generated, and also that the sequence of these tags to be known. WO 2016/162309 describes a method for spatially tagging nucleic acids of a biological specimen, including steps of (a) providing a solid support comprising different nucleic acid probes that are randomly located on the solid support, wherein the different nucleic acid probes each includes a barcode sequence that differs from the barcode sequence of other randomly located probes on the solid support; (b) performing a nucleic acid detection reaction on the solid support to locate the barcode sequences on the solid support; (c) contacting a biological specimen with the solid support that has the randomly located probes; (d) hybridizing the randomly located probes to target nucleic acids from portions of the biological specimen; and (e) modifying the randomly located probes that are hybridized to the target nucleic acids, thereby producing modified probes that include the barcode sequences and a target specific modification, thereby spatially tagging the nucleic acids of the biological specimen." Woodruff et al. (2016) Registry in a tube: multiplexed pools of retrievable parts for genetic design space exploration, Nucleic Acids Research 45: 1567-1568, provides a two-step process where a large design space is divided into deep pools of composite parts, from which individuals are retrieved and assembled to build a final construct. The pools are built via multiplexed assembly and sequenced using next-generation sequencing. Lim et al. (2018) Highly selective retrieval of accurate DNA utilizing a pool of in situ-replicated DNA from multiple next-generation sequencing platforms, Nucleic Acids Research 46:40, provides a method retrieving desired DNA from a pool of millions of DNA clones from next generation sequencing (NGS) platforms, the method involving replicating entire sequence-verified DNA molecules from NGS plates to obtain population-controlled DNA pool.

There is therefore a need to generate a large number of unique oligonucleotides with a known sequence as inexpensively and efficiently as possible.

### SUMMARY

The present disclosure described herein relates to the synthesis of oligonucleotides and/or polynucleotides. The methods described herein relate to the generation of random sequences of nucleic acids, generating oligonucleotides, where the sequence of the oligonucleotides are unknown until determined, in full or in part, after generation. In one aspect, there is provided a method for synthesizing a random oligonucleotide as set out in the claims appended hereto..

To generate a random oligonucleotide, certain embodiments synthesize nucleotides using phosphoramidite chemistry. In some embodiments, control over the phosphoramidite reaction is achieved through automated instrumentation, such as but not limited to microfluidic columns that regulate chemical reagents used in the reaction, and/or microarrays and/or microwells used to localize the reaction.

In some embodiments, spatial control is used to minimize reagents and/or materials used in the reaction when generating random oligonucleotides. Spatial control methods can be chosen by those skilled in the art, and can include but are not limited to micromirror devices used to control light-based reactions, and/or ink-jet technology used to regulate reagents used in the reaction.

In some embodiments, oligonucleotides are synthesized using an enzymatic processes, such as using a terminal deoxynucleotidyl transferase when generating random oligonucleotides. In such embodiments, enzymes can be designed to optimize the reaction and ensure that oligonucleotides are generated with randomness.

In some embodiments, relatively short random oligonucleotides are generated and then randomly combined to form longer random oligonucleotides. In some embodiments, this random combining of random oligonucleotides is a multi-step process in which multiple short random oligonucleotides are combined to form longer random oligonucleotides, which are then in turn combined to form longer oligonucleotides, and so forth in this manner until molecules of a desired size are generated.

In some embodiments, random oligonucleotides are generated to a length that is longer than desired. In such embodiments, the molecules generated are cut, digested and/or lysed to form shorter molecules.

In some embodiments, the random oligonucleotides are size-separated after they have been generated. In some of these embodiments, the randomly-generated molecules of unwanted size are discarded or re-used in previous stages in the process, such as for the random generation of more molecules.

In some embodiments, the random oligonucleotides are attached to other oligonucleotides. In these embodiments, the other oligonucleotides can serve as functional elements utilized in the application of the randomly-generated molecules. In some embodiments, the random oligonucleotides are directly generated upon other oligonucleotides. For example, a microarray comprising a set of known oligonucleotides can be used as a substrate on which oligonucleotides can be randomly-generated, resulting in molecules that have a partially known sequence and a partially unknown sequence.

In some embodiments, the random oligonucleotides that are generated are only partially random, such that, during the generation of the molecules, parts of the molecule are less-than-entirely random. In these embodiments, the less-than-entirely random aspect of the molecules being generated can be fully known, partially known, and/or partially estimated.

In some embodiments, the random oligonucleotides are filtered to remove specific unwanted molecules. These unwanted molecules can include molecules of an unwanted size and/or unwanted sequence composition, and/or any other property that is unwanted for the intended use of those molecules.

In some embodiments, the random oligonucleotides must be processed and/or modified prior to the determination of their sequence, for example, ligating them to other oligonucleotides to fit a protocol used in a given sequence technology.

To determine the nucleotide sequences of randomly-generated molecules, certain embodiments directly determine the sequence as the random oligonucleotide is generated. That is, the sequence of the randomly-generated molecule is determined, measured, or updated immediately after or shortly after one or more nucleotides are added to the molecule. In such embodiments, the reaction that adds the nucleotide could be directly observed, using for example a microwell localized reaction that generates an observable by-product of the reaction.

In some embodiments, the sequence of the randomly-generated molecule is determined after the molecule has been generated in its entirety. Determination of the sequence requires certain equipment, such as a real-time sequencer from Pacific Biosciences and/or a nanopore sequencer, which can accurately sequence individual molecules. After the sequence is determined, the molecule with a desired sequence can be recovered.

In some instances, uncertainty remains about the exact nucleotide sequence present in the randomly-generated molecule. This uncertainty can remain relatively high in comparison to the low uncertainty typically desired for most applications of poly-nucleotide sequencing.

In some embodiments, a signature of the random oligonucleotide is obtained which corresponds to the sequence of the oligonucleotide but does not represent the exact sequence of the nucleotides present in the molecule. For example, the random oligonucleotides can pass through a nanopore which can obtain a signature. This signature may be insufficient to obtain an exact sequence of the random oligonucleotide, but can nevertheless be used to identify the random oligonucleotide if it is once again sent through a similar nanopore or a similar device.

In some embodiments, the randomly-generated molecules are processed by selecting for size, using a method such as, but not limited to, column separation. In certain embodiments, oligonucleotides are screened for specific nucleotide sequences, removing oligonucleotides having unwanted sequences. In these embodiments, oligonucleotides with complimentary sequences to the unwanted oligonucleotides can be attached to a surface such as a microarray or through the use of magnetic beads attached to oligonucleotides, and this can be used to filter unwanted oligonucleotides from wanted oligonucleotides.

In some embodiments, restriction enzymes are used to digest oligonucleotides that have a specific sequence. This digestion reduces the size of the oligonucleotides, breaking them into smaller oligonucleotides. In some embodiments, these smaller oligonucleotides can be size selected and separated from the larger oligonucleotides.

In some embodiments, the randomly-generated oligonucleotides are ligated onto other oligonucleotides, using for example T-A ligation or any other method used by those skilled in the art. This ligation can be used to incorporate the random oligonucleotides into technology for other purposes.

In some embodiments, the randomly-generated oligonucleotides are single-stranded, and the complimentary strand is generated so that the final molecules are all entirely double-stranded. In some embodiments, the complimentary strand is generated using a polymerase and a random, degenerate primer. In some embodiments, the molecule that has been generated is partially double-stranded and primers are not needed to a polymerase to generate the rest of the complimentary strand.

### BRIEF DESCRIPTION OF THE DRAWINGS

Fig. 1 depicts a work flow diagram for the generation of a random oligonucleotide on a substrate as described herein.
Fig. 2 depicts a work flow diagram for the generation of random oligonucleotides from smaller oligonucleotides as described herein.
Figs. 3A-3C depict stages by which a random oligonucleotide can be generated, and its sequence subsequently determined as described herein.
Figs. 4A-4C depict stages by which a random oligonucleotide can be generated, and its sequence subsequently determined as described herein.

### DETAILED DESCRIPTION

Various embodiments are described herein, and those skilled in the art will recognize the embodiments described herein are provided only as examples. The present disclosure described herein is not limited to specific materials, reagents nor a specific process. The terminology used herein is used to describe aspects of the present disclosure required for its implementation, and is not intended to be limiting.

The technology described herein references specific instances, often using the singular form "a", "an" or "the", but reference to these instances in the singular form does not limit the present disclosure to applications in which these instances occur in isolation or alone. Those skilled in the art can determine how often these instances must occur for the application of the present disclosure, and whether these instances occur in parallel or in tandem.

As used herein, "random" or "randomly-generated" when referring to a molecule, refers to a molecule, such as an oligonucleotide and/or polynucleotide, which has a random nucleotide sequence since the molecule was generated by adding a nucleotide to the molecule at random.

As used herein, the term "oligonucleotide" refers to a molecule comprising a sequence of nucleotides, or nucleotide bases, which are linked together by some form of sugar phosphate backbone. The number of nucleotides linked together can be any number, as small as two and as large as one thousand or more. As used herein, the term "oligonucleotide" is interchangeable with the term "polynucleotide". The exact length of the nucleic acid polymer to which the term "oligonucleotide" or "polynucleotide" refers can be determined by those skilled in the art.

As used herein, the term "signature" refers to any chemical, biological or physical measurement that can be made of a molecule, which can then be used to measure that molecule in the future, with or without perfect identification.

Current methods of nucleotide synthesis have at least two qualities. First, a known sequence of nucleic acids is used to generate the desired oligonucleotide. The newly generated oligonucleotide is typically desired to be created with a very high accuracy, e.g. above 99% accuracy. Second, the generation of the oligonucleotide is generally desired to occur en masse, that is, many identical oligonucleotides are generated at the same time.

According to the present disclosure described herein, the sequence of a random oligonucleotide does not necessarily need to be known prior to generation the random oligonucleotide, provided that its sequence can be determined prior to its use. Furthermore, for some applications, such as, but not limited, to molecular cryptography as described in PCT/LTS17/058076, PCT Application Publication No. WO 2018/081113 to Sawaya, filed 24 October 2017, the exact sequence of the oligonucleotide need not be known perfectly, nor in its entirety. For some applications, only sufficient knowledge of the sequence is required to differentiate it from other random oligonucleotides. In fact, the nucleotide sequence of the oligonucleotide need not be known, as along as a "signature" of the oligonucleotide is determined prior to its use, and the signature is sufficiently distinct enough to differentiate the oligonucleotide from other random oligonucleotides.

Uniqueness of the random oligonucleotide is also required for some applications. In these applications, such as molecular cryptography (PCT/US17/058076, PCT application Publication No. WO 2018/081113), having more than one oligonucleotide with the exact same sequence is less than ideal. Although some of these applications can tolerate the presence of non-unique oligonucleotides, there is little use in having many (e.g. thousands, tens-of-thousands, or millions or more) oligonucleotides with the exact same sequence. Hence, the present disclosure takes a unique approach, and resolves unique challenges, in comparison to contemporary methods of nucleotide synthesis.

The present disclosure generates unique, random oligonucleotides by: a) generating at least one molecule comprising nucleic acids by adding at least one nucleotide to the molecule at random, wherein the molecule generated is a random oligonucleotide; b) determining the nucleotide sequence of the random oligonucleotide; and c) selecting random oligonucleotides using certain characteristics of the random oligonucleotides. In certain embodiments, selecting random oligonucleotides is part of processing the oligonucleotides to prepare them for various uses. In certain embodiments, the molecules are measured. In certain embodiments, the random oligonucleotide can be identified using a similar or identical measuring technique used to measure the oligonucleotide.

The synthesis of molecules and oligonucleotides to generate the molecules as described herein can be achieved by a range of methods known to those skilled in the art. These methods can include, but are not limited to, phosphoramidite chemistry and/or enzymatic-based synthesis.

A wide array of methods are available for oligonucleotide synthesis as described in Hughes and Ellington (2017) "Synthetic DNA synthesis and assembly: putting the synthetic in synthetic biology". Perspect. Biol.; 9: a023812; and described in Kosuri and Church (2014) "Large-scale de novo DNA synthesis: technologies and applications." Nature Methods; 11:499-507. These methods can be utilized on their own, or combined, to generate random oligonucleotides. The methods for generating random oligonucleotides described herein are examples that can be utilized in this disclosure, but the present disclosure is not limited to the specific the oligonucleotide synthesis methods. As the state of the art of oligonucleotide synthesis develops, alternative methods of oligonucleotide synthesis can be utilized in the generation of random oligonucleotides in the present disclosure.

Currently, large-scale, low-cost nucleotide synthesis occurs using phosphoramidite chemistry on microarray synthesizers. Synthesis with this method allows multiple oligonucleotides to be generated in parallel with high sequence accuracy. Details about these methods are described in Heller (2002) DNA microarray technology: devices, systems, and applications. Annu Rev Biomed Eng., 4:129-53; and LeProust et al., (2010) Synthesis of high-quality libraries of long (150mer) oligonucleotides by a novel depurination controlled process. Nucleic Acids Res. 38(8): 2522-40.

In certain embodiments, oligonucleotides having a specific sequence are synthesized in parallel on a microarray or in microwells using phosphoramidite synthesis (with current techniques such as but not limited to LeProust above; and U.S. Patent Nos. 6,458,583 to Bruhn et al.; 7,544,793 to Gao et al.; 9,555,388 to Banyai et al., and these identical oligonucleotides serve as the substrate on which random oligonucleotides are then generated.

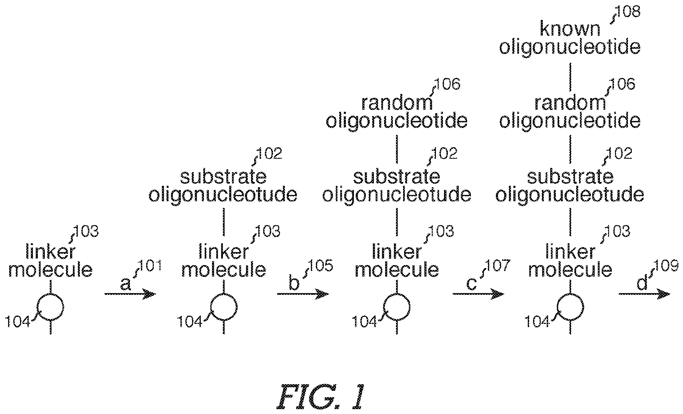

Referring to Figure 1, step **101** generates a substrate oligonucleotide **102** on a linker molecule **103,** that may be linked to, for example, but not limited to, a microarray or a magnetic bead **104.** In step **105,** a random oligonucleotide **106** is generated on substrate oligonucleotide **102.** In step **107,** a non-random or known oligonucleotide **108** is generated on the growing molecule. Step **109** can either be a processing of the generated molecule for use, or repeating step **105** or step **107** in any combination, which can be repeated as many times as desired.

The substrate oligonucleotides **102** can, in some embodiments, have properties that facilitate future use of the random oligonucleotides. For example, the substrate oligonucleotides **102** can comprise an appropriate sequence that can act as a primer for nucleotide amplification in a polymerase chain reaction (PCR) protocol. These substrate oligonucleotides **102** can also, in some embodiments, serve as indexes to help identify the oligonucleotides being generated. Furthermore, these substrate oligonucleotides **102** can also, in some embodiments, act as adapters, or serve other functions not limited to the functions discussed here. The generated random oligonucleotides **106** that are connected to the oligonucleotides of a known sequence can, in some embodiments, then act as substrates for the generation of oligonucleotides with a specific, known sequence **108.** Following this, in some embodiments, more random oligonucleotides **106** can also be generated on this molecule, and in some embodiments, then more oligonucleotides of known sequence can be generated, and so forth in this manner, alternating between the generation of known and unknown, random sequence moieties until a desired molecule is generated.

After a group of identical oligonucleotides are synthesized in tandem on a microarray, to serve as substrates for synthesis of random oligonucleotides, in some embodiments, random oligonucleotide synthesis occurs upon these substrate oligonucleotides **102** using phosphoramidite synthesis. In contrast to traditional synthesis on microarrays, the random synthesis used to generate random oligonucleotides **106** is undirected, or in some embodiments, partially directed such that the exact sequence being generated is completely unknown, or when partially directed, partially unknown. As used herein, "partially directed", refers to adding a selected nucleotide or oligonucleotide to an oligonucleotide in a manner that is not entirely random.

In some embodiments, random oligonucleotides are synthesized using a protein that enzymatically synthesizes DNA, such as a terminal deoxynucleotidyl transferase (TdT). In some embodiments, these protein enzymes are specifically designed to synthesize random oligonucleotides, so that they do not synthesize specific oligonucleotides dependent on any sequence moiety of the random oligonucleotide that has previously been generated by the enzyme.

In some embodiments, a random oligonucleotide is synthesized within a microwell in a system similar to, but not limited to, U.S. Patent Nos. 9,845,501 to Williams; and 7,302,146 to Turner et al.. In such embodiments, the synthesis of the random oligonucleotide includes the use of labelling molecules, such as the labelling techniques discussed within U.S. Patent Nos. 9,845,501 to Williams; and 8,580,539 to Korlach. The labelling of the molecules serve to determine the sequence of the random oligonucleotide being synthesized immediately after or shortly after a nucleotide is added to the random oligonucleotide. In these embodiments, the random oligonucleotide being generated by this process is only of an unknown sequence for a short period of time. The newly added nucleotide becomes known and the sequence of the oligonucleotide is thus determined. In these embodiments, the newly generated oligonucleotide becomes the substrate oligonucleotide on which another random oligonucleotide or nucleotide is chemically bonded. The sequence is then determined after a random oligonucleotide or a nucleotide is added. This continues in this manner until a random oligonucleotide of approximately-known length and approximately-known or fully-known sequence has been generated. In such embodiments, the methods alternate between (a) randomly generating an oligonucleotide, and (b) determining the sequence of that oligonucleotide. The method for determining the sequence of the oligonucleotide in such embodiments is further detailed below discussing methods for determining sequence composition of the randomly-generated oligonucleotides.

An entirely undirected synthesis of oligonucleotides can lead to some random oligonucleotides being generated that may be problematic for the future use of these oligonucleotides. For example, homopolymer runs, such as oligonucleotides with a "CCCC" sequence of nucleotides, can lead to non-specific annealing and/or problems with sequencing on some sequencing platforms (see Xu et al, (2009) Design of 240,000 orthogonal 25mer DNA barcode probes, PNAS, Vol. 107 No. 7 pp. 2289-2294). To avoid these and other less-than-ideal random oligonucleotides prior to their synthesis, reagents used in the reactions can be partially directed in certain embodiments, using ink-jet technology, for example as described in US Patent Nos. 6,221,653 to Caren et al.; 6,476,215 to Okamoto et al; and U.S. Patent Nos. 6,077,674 to Schleifer et al.; and 7,572,907 to Dellinger et al..

In certain embodiments, the reactions can also be partially directed using micromirrors to regulate light-controlled reactions, such as those described in U.S. Patent Nos. 6,545,758 to Sandstrom and 7,157,229 to Cerrina et al.. An example of partial control over random synthesis of oligonucleotides would be to limit the quantity of a given nucleotide base. For example, if a run of homopolymer cytosines were less-than-ideal for a given application, then nucleotide bases other than cytosine could be favorably directed, using, for example, ink-jet technology, in the synthesis of the random oligonucleotide. Furthermore, light controlled reactions can be modified with micromirrors. Using light controlled reactions, specific nucleic acid bases can be favored or disfavored depending on the nucleotide bases that are estimated or known to be present in the solution in proximity to the reaction.

In certain embodiments, after a random oligonucleotide is generated, a non-random oligonucleotide or nucleotide is attached to the random oligonucleotide, either by directly generating on the random oligonucleotide, and/or attaching a non-random oligonucleotide to the random oligonucleotide by ligation.

In certain embodiments, random oligonucleotides are generated in a multi-step method. The method comprises generating at least one shorter random oligonucleotide. The shorter random oligonucleotides are then combined to generate longer random oligonucleotides. The longer random oligonucleotides can be then can be combined to generate even longer oligonucleotides. The random nucleotides are combined, for example, through ligation using an enzyme or molecular bonding directed by phosphoramidite chemistry, to generate longer oligonucleotides. In certain embodiments, a filtration step is used to remove unwanted random oligonucleotides at one or more stages of the random oligonucleotide generation. In certain embodiments, the multi-step method comprises: generating a random oligonucleotide by adding one nucleotide to another nucleotide at random; combining a first random oligonucleotide to another random oligonucleotide at random; screening the random oligonucleotides for certain characteristics; removing random oligonucleotides with certain characteristics; and combining the remaining random oligonucleotides at random. The screening, removing, and combining steps can be repeated to create random oligonucleotides with desired characteristics.

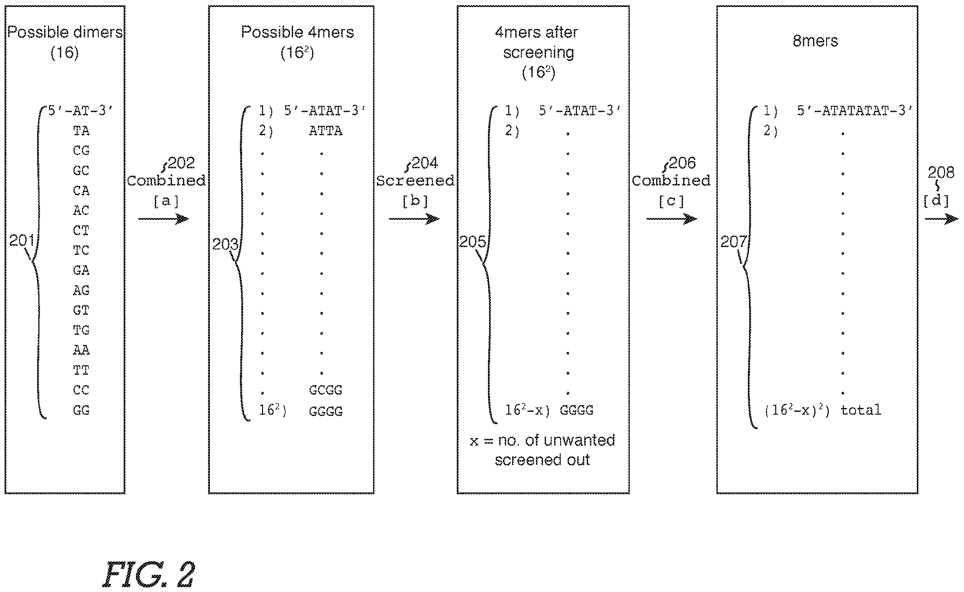

Referring to Figure 2, in certain embodiments, random oligonucleotides comprise dimers or two-mers. A Dimer **202,** is an oligonucleotide comprising two nucleotides. Given there a four different types of nucleic acid bases, A= adenosine, T= thymidine, C = cytosine, and G = guanine, there are 16 different combinations of two nucleotides. Thus, dimers can be one of sixteen different sequences: AT, TA, CG, GC, CA, AC, CT, TC, GA, AG, GT, TG, AA, TT, CC, GG.

A four-mer **203** is an oligonucleotide comprising four nucleotides. A four-mer oligonucleotide has 16² combinations of nucleic acid bases. If four-mer oligonucleotides are generated by combining dimer oligonucleotides, the four-mer oligonucleotides can, for example, be screened for homopolymer oligonucleotides in certain embodiments. A homopoymer oligonucleotide comprises a single type of nucleotide base, e.g. AAAA, TTTT, CCCC, and GGGG. This screening can occur by a process such as, but not limited to, washing the four-mer oligonucleotides over a microarray of oligonucleotides with terminal sequences that are themselves homopolymer oligonucleotides, thus complimentary to the less-than-ideal, randomly-generated homopolymer oligonucleotides. In certain embodiments, the dimers are random dimers and the four-mers are random four-mers.

In certain embodiments, screening for the unwanted oligonucleotides occurs during the generation of the random oligonucleotides. Screening occurs by using oligonucleotides that have complimentary sequences to the unwanted oligonucleotides, as filters that bind to the unwanted oligonucleotides to remove them from a solution of random oligonucleotides.

An exemplary embodiment, as seen in Figure 2, Dimers **201** are randomly combined **(202)** to generate four-mer oligonucleotides **203.** There is 16² possible resulting four-mer oligonucleotides. The four-mer oligonucleotides are then screened **(204)** for unwanted oligonucleotides. The number of remaining four-mers is 16² - x, where x = number of unwanted oligonucleotides screened out. The remaining four-mers **205** are then combined **(206)** to generate eight-mers **207,** which are oligonucleotides comprising 8 nucleotides. The number of eight-mers is (16² - x)²) where x = number of unwanted oligonucleotides screened out. Step **208** is repeating a screening step, repeating a combination step, or repeating any combination of the repeating step or the screening step. Step **208** can be repeated as many times as is desired.

In certain embodiments, random oligonucleotides are chemically bonded with oligonucleotides of a known sequence. These known oligonucleotides can, in some embodiments, be linked to the 3' and/or 5' end of the random oligonucleotides. In some embodiments, this process occurs multiple times, resulting in oligonucleotides that contain a combination of random oligonucleotides and oligonucleotides of a known sequence.

In some embodiments, after a random oligonucleotide has been generated, they can be filtered for unwanted oligonucleotides. The filtering for unwanted oligonucleotides can occur before or after the random nucleotide has been linked physically with other oligonucleotides. The other oligonucleotides can have a known sequence, a partially random sequence, or entirely unknown sequence. This filtration can occur by removing unwanted oligonucleotides. Unwanted oligonucleotides can be removed, for example, by filtering the generated oligonucleotides based on size, using for example, column-based size separation techniques.

In certain embodiments, removing unwanted oligonucleotides can occur in combination with other filtration processes, such as filtering for unwanted sequences. Filtering for unwanted sequences can be achieved by any of the following methods or combination thereof: binding of unwanted oligonucleotides to complimentary oligonucleotides bound to a surface or bead, and subsequently washing the desired oligonucleotides away from the unwanted oligonucleotides; binding of unwanted oligonucleotides to complimentary oligonucleotides, which are bound to proteins which can facilitate degradation of unwanted oligonucleotides; by using restriction endonucleases to cleave unwanted oligonucleotides, thus reducing their size allowing for subsequent filtration by size to remove the unwanted oligonucleotides; and/or using any other method that is known by those skilled in the art to filter out oligonucleotides having unwanted sequences.

To determine the nucleotide sequence of the generated random oligonucleotides, a range of sequencing techniques can be used, individually or in combination. Those skilled in the art can determine the best, preferred sequencing technique(s), and as sequencing technology advances, those techniques may be utilized for determining sequences according to the present disclosure. Importantly, sequencing techniques that are used to determine the nucleotide sequences of the random oligonucleotides must either allow the random oligonucleotides to be collected for use after they have been sequenced, or alternatively, generate a copy or complimentary oligonucleotide that can be collected after the random oligonucleotide has been sequenced. This requirement allows the generated random oligonucleotides to be used after their sequence has been determined.

In certain embodiments, the random oligonucleotide must be processed prior to the determination of its sequence. This processing can include, but is not limited to, addition of nucleotides to the random oligonucleotide, the removal of specific oligonucleotides based on their size or sequence, and/or changing the solution in which the random oligonucleotides exist, as those skilled in the art can appreciate. Specific methods for processing random oligonucleotides to prepare them for sequencing depend on the sequencing method to be used. As methods for sequencing chains of nucleic acids change, processing the random oligonucleotides to prepare them for sequencing may also change, and those skilled in the art can determine the appropriate methods for processing the random oligonucleotides to prepare them for sequencing.

In certain embodiments, random oligonucleotides need to be collected after sequencing. A potential option for sequencers that allow for the collection of nucleotides after sequencing are the Pacific Biosciences single-molecule real-time sequencers, for example discussed in Eid et al. (2009) "Real-Time DNA Sequencing from Single Polymerase Molecules", Science 323(5910):133-38. The sequencers described therein observe a polymerase replicating a polynucleotide in real-time using indicator molecules that can uniquely identify nucleotides as they are incorporated into a polynucleotide as it is being replicated.

The result is less-than-ideal for some situations for which the present disclosure can be used, as extraction of these polynucleotides after they have been sequenced will result in more than one copy of the polynucleotides that were originally sequenced. For example, if the randomly-generated oligonucleotides are to be used in molecular cryptography, such as described in Application PCT/US17/058076 WO Publication WO 2018/081113, then duplicate oligonucleotides are less than ideal. Although the synthesized polynucleotide could, in some embodiments, be separated from the original polynucleotides using an attachment of a bead to the polynucleotide or oligonucleotide being sequenced, other approaches may provide a better yield.

In some embodiments, an approach to separate the synthesized polynucleotide from the original polynucleotide would be to have randomly-generated a single-stranded polynucleotide or oligonucleotide, or partially single-stranded polynucleotide. In such embodiments the copy of the molecule generated during sequencing would be the compliment to the randomly-generated oligonucleotide. Assuming that the molecule being sequenced does not contain a hair-pin loop (such as is currently used in Pacific Biosciences sequence preparation, U.S. Patent No. 9,404,146 to Travers et al.), the newly synthesized polynucleotide and the original, complimentary molecule would be a randomly-generated double-stranded oligonucleotide with a known sequence that can be extracted from the sequencer.

In certain embodiments, sequencing technology can be utilized to directly generate a random oligonucleotide and then immediately determine its sequence. Sequencing technology observes the reaction between a polymerase and polynucleotide by observing indicator molecules, which indicate that a given nucleotide base has been incorporated into the polynucleotide to be sequenced. In certain embodiments, the reaction is ideally directed by an enzyme, such as a TdT, that extends the oligonucleotide that is being generated randomly.

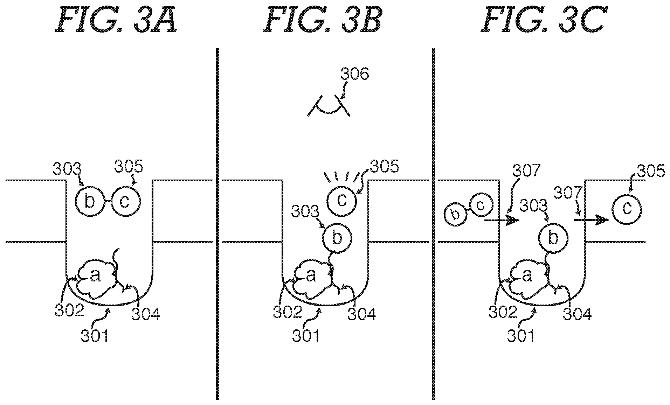

Referring to Figure 3A-C, in some embodiments, the reaction occurs in in a microwell **301** which contains an enzyme "a" **302.** In certain embodiments, enzyme **302** is TdT.

Referring to Figure 3B, in certain embodiments, enzyme **302** ("a"), and immediately after or shortly after a nucleotide "b" **303** has been randomly added to the oligonucleotide **304,** the indicator molecule "c" **305** can be observed **306.**

Referring to Figure 3C, in certain embodiments, reagents are added to **307** and removed from the microwell **301** using microfluidics.

In some embodiments, the addition of a new nucleotide to the oligonucleotide is added at random because the solution in the proximity of the enzyme contains an approximately equal quantity of various nucleotide bases. In other embodiments, the solution surrounding the enzyme that is synthesizing the random oligonucleotide does not contain an equal proportion of nucleotide bases and thus the composition of the oligonucleotide, while still random, is not expected to have equal proportions of each nucleotide base. In some embodiments, the solution surrounding the enzyme is directed to contain specific ratios of nucleotide bases in order to direct the generation of the random oligonucleotide. This direction can, for example, favor the incorporation of certain nucleotide bases over others to control the composition of the random oligonucleotide.

Another sequencing technology that has the ability to recover an oligonucleotide after its sequence has been determined are nanopore sequencers, such as those described in Loman and Watson (2015) "Successful test launch for nanopore sequencing" Nature Methods volume 12, pages 303-304. In certain embodiments, the random oligonucleotide is generated in its entirety, and then the sequence is determined using a nanopore sequencer that directly determines the random oligonucleotide's sequence after which the oligonucleotide is recovered.

In other embodiments, nanopore technology is used to observe the incorporation of a random nucleotide to an oligonucleotide by using an indicator molecule, similar but not identical to Fuller et al. (2016), "Real-time single-molecule electronic DNA sequencing by synthesis using polymer-tagged nucleotides on a nanopore array," PNAS, May 10, 2016, Vol. 113 No. 19, pp. 5233-5238 (available at www.pnas.org/cgi/doi/10.1073/pnas.1601782113).

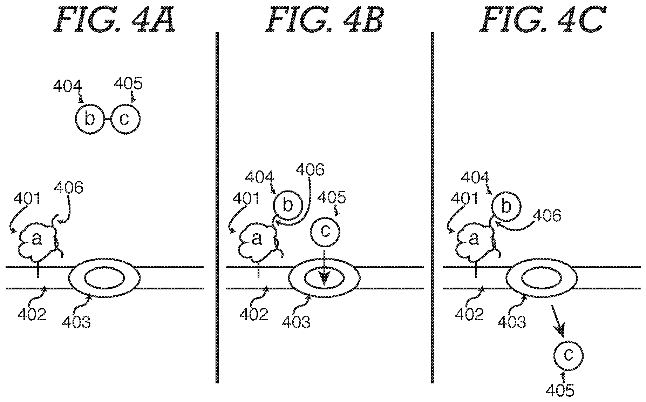

Figures 4A-C illustrates a real-time single-molecule electronic DNA sequencing by synthesis using polymer-tagged nucleotides on a nanopore array. This allows observation of the generation of a random oligonucleotide, and its sequence is determined shortly after or immediately after its generation.

Referring to Figure 4A, molecule **401** is attached to a membrane **402** in close proximity to a nanopore **403.** A random oligonucleotide **406** is attached to the molecule **401.** In certain embodiments, molecule **401** is an enzyme TdT.

Referring to Figures 4B-C, after molecule "a" **401** adds a nucleotide **404** "b", at random to the random oligonucleotide **406,** the indicator molecule "c" **405** is free to pass through the nanopore **403,** generating a signal.

In some embodiments, the nucleotide bases in the reaction solution need to be regulated to ensure that the sequence of oligonucleotide being generated is entirely random. With an entirely random sequence, the presence of one nucleotide base at any given position in the random oligonucleotide cannot be used to predict the presence or absence of a nucleotide base at another position in the random oligonucleotide.

In embodiments where the sequence of the oligonucleotide is determined immediately after or shortly after the oligonucleotide has been generated, or determined when the oligonucleotide is currently being generated, the solution around this generation may, in some embodiments, be controlled. In some embodiments, a microfluidic control mechanism is used, in which microchannels regulate the reactive solution, allowing the solution to flow across the reactive area or microwell. In some embodiments, ink-jet technology is utilized to regulate the reactive solution in real-time. In some embodiments, a flow of solution containing nucleotide bases in a specific concentration is washed across the reactive surface or microwell. In some embodiments, directed energy, such as heat or light-based reactions are controlled using micromirrors and/or other forms of control over the direction of energy that influences the reaction.

In some embodiments, the exact sequence of the random oligonucleotide is not determined with certainty. Knowledge of the exact sequence of the random oligonucleotide may not be necessary for some uses of the random oligonucleotide. For some uses of the random oligonucleotide, imperfect information about the sequence of a given random oligonucleotide is sufficient to differentiate that oligonucleotide from other randomly-generated oligonucleotides. Therefore, in some embodiments, partial sequence information, or partially inaccurate sequence information is obtained from the randomly-generated oligonucleotides. In some embodiments, a "signature" of the random oligonucleotide is obtained from a sequencer, which can then be used to identify a randomly-generated oligonucleotide and/or differentiate it from other random oligonucleotides. This signature can be, for example but not limited to, the electrical signal obtained from passage of the random oligonucleotide through a pore embedded in a membrane, or the kinetic signature of the molecule obtained from its interaction with another molecule or enzyme. The signature obtained from a given sequencing method can then be used to identify the random oligonucleotide. For example, the random oligonucleotide can be identified in a protocol where the random oligonucleotide is used as an identifier for another oligonucleotide that has been ligated to the random oligonucleotide, when sequencing the combined oligonucleotide on the same or similar sequencing platform.

Methods for processing the randomly-generated molecules.

The random oligonucleotides generated by the method of the present disclosure can be used in various technologies, such as molecular cryptography, (PCT Application No. PCT/US17/058076 or other methods that require molecular level identification such as methods described in U.S. Patent Pub. Nos. 2015/0211050; and 2015/0211061. The method of processing the randomly-generated molecules depends on the technology using the randomly-generated molecules.

In some embodiments, the random oligonucleotides are processed prior to the determination of their sequence by selecting certain oligonucleotides having certain characteristics. The processing allows efficient sequencing, and the nucleotide sequences generated by the sequencer are accurate and generated only for oligonucleotides that are of use. However, in some embodiments, it can also desired to also process the oligonucleotides after their sequence has been determined. This would be required when, for example, the sequence of the oligonucleotide being generated is determined immediately after or shortly after its generation. This may also be required when generating a large random oligonucleotide and its sequence is subsequently determined, and this large random oligonucleotide must be processed into smaller oligonucleotides prior to use.

In some embodiments, random oligonucleotides are processed by selecting for size, using a selection technique such as, but not limited to, column separation. Molecules of an unwanted size can be separated from molecules of a wanted size, choosing molecules that contain a specific number of nucleotide bases when desired.

In some embodiments, random oligonucleotides are screened for specific sequences by removing oligonucleotides that have less-than-ideal and/or unwanted sequences. In these embodiments, random oligonucleotides with complimentary nucleotide sequences to the unwanted oligonucleotides can be attached to a surface such as a microarray or through the use of magnetic beads attached to random oligonucleotides. The random oligonucleotide can then be introduced to this surface by washing them over the microarray, or introduced to a solution containing magnetic beads. The desired random oligonucleotides remain in solution after the solution has been washed over the microarray, or remain in solution when magnetic beads are removed. Those skilled in the art can determine the best techniques to screen for specific oligonucleotides sequences to avoid the presence of these unwanted molecules in a final mixture.

In some embodiments, restriction enzymes are used to digest random oligonucleotides that have a specific sequence. This digestion reduces the size of the oligonucleotides, breaking them into smaller oligonucleotides. These smaller molecules can be size selected and separated from the larger oligonucleotides.

In some embodiments, the random oligonucleotides are ligated and/or otherwise attached onto other oligonucleotides, using for example T-A ligation or any other method used by those skilled in the art. This ligation can be used to incorporate the random oligonucleotides into technology for other purposes, for example purposes disclosed in U.S. Patent Pub. Nos. 2015/0211050; and 2015/0211061.

In some embodiments, the generated random oligonucleotides are single-stranded. In some embodiments, the process by which the sequence of the random oligonucleotide is determined results in single-stranded random oligonucleotides. If the sequenced random oligonucleotides are entirely single-stranded and their desired use requires them to be double-stranded, then in some embodiments, a technique is used to generate a strand with a complimentary sequence. One such technique may be the use of a DNA polymerase along with random, degenerate primers to generate matching strands. Random primers are not always necessary. In some embodiments, the randomly-generated single-stranded oligonucleotides are attached to a double-stranded DNA molecule prior to or during the determination of their sequence. In certain embodiments, the randomly-generated single-stranded oligonucleotides are ligated to a double-stranded DNA molecule after their sequence has been determined. In some embodiments, the random oligonucleotides are attached to molecules with a known sequence moiety, for which specific primers can be designed to generate complimentary strands using a polymerase.

In some embodiments, after the random oligonucleotides are processed into their desired form, they can be packaged and this package can be labelled with the sequences present in the solution. In some embodiments, when the randomly-generated molecules are to be used in molecular cryptography (see PCT/US17/058076), the exact sequences and/or signatures of the molecules in the solution must be kept secure, and in these embodiments the information about the sequences present are not packaged with the solution of the molecules and can instead, be sent in a separate package and/or have the data file containing the sequence information be securely delivered to the appropriate parties.

## Claims

1. A method for synthesizing a random oligonucleotide, the method comprising:
(a) synthesizing at least one random oligonucleotide comprising nucleotides by adding one or more nucleotides at random to the oligonucleotide;
(b) determining the sequence of the random oligonucleotide; and
(c) selecting random oligonucleotides based on specific sizes of the random oligonucleotides.

2. The method of claim 1, wherein the random oligonucleotides are synthesized using phosphoramidite chemistry.

3. The method of claim 1, wherein the random oligonucleotides are synthesized using an enzymatic process.

4. The method of claim 1, wherein the random oligonucleotides are synthesized within a microwell or on a microarray.

5. The method of claim 1, wherein the random oligonucleotides are synthesized having a sequence that is partially known.

6. The method of claim 3, wherein an indicator molecule becomes reactive after a nucleotide is added to the molecule using the enzymatic process.

7. The method of claim 1, wherein adding a nucleotide to the molecules is partially directed.

8. The method of claim 1, wherein microfluidics or directed energy are used to control reaction conditions.

9. The method of claim 1, wherein the sequence of the random oligonucleotide is determined using a nanopore sequencer.

10. The method of claim 1, wherein the sequence of the random oligonucleotide is determined to identify the random oligonucleotide.

11. The method of claim 1, further comprising:
(d) identifying unwanted random oligonucleotides;
(e) screening the random oligonucleotides by removing the unwanted random oligonucleotides with specific sequences of nucleotides or sizes; and
(f) combining the screened random oligonucleotides at random.

12. The method of claim 11, further comprising repeating any of steps (d)-(f) to create random oligonucleotides with specific sequences of nucleotides or sizes of nucleotides.

## Patentansprüche

1. Verfahren zum Synthetisieren eines zufälligen Oligonukleotids, wobei das Verfahren Folgendes umfasst:
(a) das Synthetisieren mindestens eines zufälligen Oligonukleotids, das Nukleotide umfasst, durch zufälliges Zusetzen eines oder mehrerer Nukleotide zu dem Oligonukleotid;
(b) das Bestimmen der Sequenz des zufälligen Oligonukleotids; und
(c) das Wählen von zufälligen Oligonukleotiden auf der Basis spezifischer Größen der zufälligen Oligonukleotide.

2. Verfahren nach Anspruch 1, wobei die zufälligen Oligonukleotide unter Anwendung von Phosphoramiditchemie synthetisiert werden.

3. Verfahren nach Anspruch 1, wobei die zufälligen Oligonukleotide unter Anwendung eines enzymatischen Verfahrens synthetisiert werden.

4. Verfahren nach Anspruch 1, wobei die zufälligen Oligonukleotide innerhalb einer Mikrovertiefung oder auf einem Mikroarray synthetisiert werden.

5. Verfahren nach Anspruch 1, wobei die zufälligen Oligonukleotide so synthetisiert werden, dass sie eine teilweise bekannte Sequenz aufweisen.

6. Verfahren nach Anspruch 3, wobei ein Indikatormolekül reaktiv wird, nachdem ein Nukleotid zu dem Molekül unter Anwendung des enzymatischen Verfahrens zugegeben worden ist.

7. Verfahren nach Anspruch 1, wobei das Zugeben eines Nukleotids zu den Molekülen teilweise gezielt ist.

8. Verfahren nach Anspruch 1, wobei Mikrofluidik oder gezielte Energie zum Regulieren der Reaktionsbedingungen verwendet werden.

9. Verfahren nach Anspruch 1, wobei die Sequenz des zufälligen Oligonukleotids unter Anwendung eines Nanopore-Sequenzers bestimmt wird.

10. Verfahren nach Anspruch 1, wobei die Sequenz des zufälligen Oligonukleotids bestimmt wird, um das zufällige Oligonukleotid zu identifizieren.

11. Verfahren nach Anspruch 1, ferner Folgendes umfassend:
(d) das Identifizieren unerwünschter zufälliger Oligonukleotide;
(e) das Screenen der zufälligen Oligonukleotide durch Entfernen der unerwünschten zufälligen Oligonukleotide mit spezifischen Sequenzen von Nukleotiden oder Größen; und
(f) das zufälliges Kombinieren der gescreenten zufälligen Oligonukleotide.

12. Verfahren nach Anspruch 11, ferner das Wiederholen irgendwelcher der Schritte (d)-(f) umfassend, um zufällige Oligonukleotide mit spezifischen Sequenzen von Nukleotiden oder Größen von Nukleotiden zu erzeugen.

## Revendications

1. Procédé de synthèse d'un oligonucléotide aléatoire, le procédé comprenant :
(a) la synthèse d'au moins un oligonucléotide aléatoire comprenant des nucléotides par l'ajout aléatoire d'un ou de plusieurs nucléotides à l'oligonucléotide ;
(b) la détermination de la séquence de l'oligonucléotide aléatoire ; et
(c) la sélection d'oligonucléotides aléatoires sur la base de tailles spécifiques des oligonucléotides aléatoires.

2. Procédé selon la revendication 1, dans lequel les oligonucléotides aléatoires sont synthétisés en utilisant une chimie de phosphoramidite.

3. Procédé selon la revendication 1, dans lequel les oligonucléotides aléatoires sont synthétisés en utilisant un processus enzymatique.

4. Procédé selon la revendication 1, dans lequel les oligonucléotides aléatoires sont synthétisés dans un micropuits ou sur un microréseau.

5. Procédé selon la revendication 1, dans lequel les oligonucléotides aléatoires sont synthétisés en ayant une séquence qui est partiellement connue.

6. Procédé selon la revendication 3, dans lequel une molécule indicatrice devient réactive après qu'un nucléotide est ajouté à la molécule en utilisant le processus enzymatique.

7. Procédé selon la revendication 1, dans lequel l'ajout d'un nucléotide aux molécules est partiellement dirigé.

8. Procédé selon la revendication 1, dans lequel la microfluidique ou une énergie dirigée sont utilisées pour réguler les conditions de réaction.

9. Procédé selon la revendication 1, dans lequel la séquence de l'oligonucléotide aléatoire est déterminée en utilisant un séquenceur par nanopores.

10. Procédé selon la revendication 1, dans lequel la séquence de l'oligonucléotide aléatoire est déterminée pour identifier l'oligonucléotide aléatoire.

11. Procédé selon la revendication 1, comprenant en outre :
(d) l'identification d'oligonucléotides aléatoires non voulus ;
(e) le criblage des oligonucléotides aléatoires par le retrait des oligonucléotides aléatoires non voulus avec des séquences de nucléotides ou des tailles spécifiques ; et
(f) la combinaison aléatoire des oligonucléotides aléatoires criblés.

12. Procédé selon la revendication 11, comprenant en outre la répétition de n'importe laquelle des étapes (d)-(f) pour créer des oligonucléotides aléatoires avec des séquences de nucléotides ou des tailles de nucléotides spécifiques.
